# EUROPÄISCHE PATENTSCHRIFT

(11) **EP 2 265 567 B1**
(45) Veröffentlichungstag und Bekanntmachung des Hinweises auf die Patenterteilung: **01.08.2018**
(21) Anmeldenummer: 09723169.0
(22) Anmeldetag: 13.01.2009
(51) Int. Cl.: B01D 17/02, C07C 57/065, C07C 57/07, B01J 19/24, C07C 57/075

(54) **VERFAHREN ZUR AUFREINIGUNG VON METHACRYLSÄURE**
METHOD FOR PURIFYING METHACRYLIC ACID
PROCÉDÉ D'ÉPURATION D'ACIDE MÉTHACRYLIQUE

(30) Priorität: 20.03.2008 DE 102008000787
(43) Veröffentlichungstag der Anmeldung: 29.12.2010
(73) Patentinhaber: Evonik Röhm GmbH, 64293 Darmstadt (DE)
(72) Erfinder: GROPP, Udo, 83093 Bad Endorf (DE); SOHNEMANN, Stefanie, 51491 Overath (DE); PROTZMANN, Guido, 64625 Bensheim (DE); MERTZ, Thomas, 64625 Bensheim (DE)
(86) Internationale Anmeldenummer: PCT/EP2009/050295
(87) Internationale Veröffentlichungsnummer: WO 2009/115350

(56) Entgegenhaltungen:
- EP-B1- 2 167 455
- GB-A- 1 100 370
- US-A- 3 320 305
- US-A- 5 041 646

## Beschreibung

Die vorliegende Erfindung betrifft ein Verfahren zur Aufreinigung von Methacrylsäure.

Verfahren zur Herstellung von Methacrylsäure sind seit langem bekannt. Eine übliche Verfahrensweise besteht in der kontrollierten Oxidation von Kohlenwasserstoffgasen, beispielsweise Propylen oder Butylen. Nachteilig an diesen Verfahren sind die insgesamt gesehen eher geringen Ausbeuten, die hierdurch erhalten werden.

Darüber hinaus kann Methacrylsäure durch die Umsetzung von Methacrylamid mit Wasser erhalten werden. Dieses Verfahren wird insbesondere in US 7,253,307 beschrieben. Gemäß dieser Druckschrift kann die Umsetzung des Methacrylamids mit Wasser in einem Rührkesselreaktor oder einem Röhrenreaktor erfolgen. Vorzugsweise wird die Reaktion bei einem Druck im Bereich von 3,65 bis 7,70 bar und einer Temperatur im Bereich von 50 bis 210 °C durchgeführt. Die erhaltene Reaktionsmischung wird durch einen Wärmetauscher abgekühlt und in einen Phasentrenner geleitet. Hierbei wird eine wässrige Phase von einer organischen Phase getrennt. Das gewünschte Produkt wird aus der organischen Phase anschließend isoliert.

Die in US 7,253,307 beschriebenen Verfahren zur Herstellung von Methacrylsäure führen bereits zu guten Ausbeuten bei einer hohen Reinheit. Allerdings stellt Methacrylsäure ein wichtiges Erzeugnis der chemischen Industrie dar, welches als Ausgangsstoff für viele wichtige Produkte dient. Daher ist eine maximale Ausbeute, eine besonders hohe Reinheit bei geringen Herstellungskosten wesentlich für den wirtschaftlichen Erfolg eines Herstellungsprozesses für ein derart wichtiges Produkt. Schon relativ kleine Verbesserungen hinsichtlich der Ausbeuten, der Standzeiten der Anlagen oder ähnlicher Verfahrensmerkmale führen zu einem bedeutenden Fortschritt hinsichtlich der Abfallmengen und der Herstellkosten.

Die GB 1100370 offenbart ein Verfahren zur Herstellung von Methacrylsäure durch Umsetzung von Methacrylnitril mit Schwefelsäure in einem Rundkolben. Die Umsetzung erfolgt im molaren Verhältnis von Methacrylnitril zu Schwefelsäure von 1:1.

US 5041646 offenbart ein Verfahren zur Herstellung von Z-2-Methyl-2-butensäure durch schwefelsaure Hydrolyse von 2-Methyl-2-butennitril in einem Reaktor.

Die US 3320305 beschreibt ein Verfahren zur Herstellung von Carbonsäuren durch Umsetzung von gesättigten oder ungesättigten Nitrilen in Schwefelsäure mit einem nitrosierenden Agent.

GB 1100370, US 5041646 US 3320305 und offenbaren nicht die Umsetzung von Methacrylamid mit Wasser.

Für den Fachmann ist es nicht naheliegend, den Reaktant Methacrylnitril gegen Methacrylamid auszutauschen. Auch der beschriebene Einsatz von Schwefelsäure erfordert eine spezielle Ausgestaltung des Verfahrens und der Anlagenteile.

In Anbetracht des Standes der Technik ist es nun Aufgabe der vorliegenden Erfindung ein Verfahren zur Herstellung oder zur Aufreinigung von Methacrylsäure zur Verfügung zu stellen, das besonders einfach und kostengünstig durchgeführt werden kann. Hierbei sollten insbesondere die Ausbeute, die Reinheit und die Standzeit der Anlage zur Herstellung von Methacrylsäure erhöht werden. Weiterhin sollte die Bildung von unerwünschten Nebenprodukten minimiert werden. Des Weiteren war es mithin Aufgabe der vorliegenden Erfindung eine Anlage zur Herstellung von Methacrylsäure bereitzustellen, die eine möglichst kostengünstige Produktion von Methacrylsäure ermöglicht.

Gelöst werden diese sowie weitere nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen des erfindungsgemäßen Verfahrens werden in Unteransprüchen unter Schutz gestellt.

Gegenstand der vorliegenden Erfindung ist dementsprechend ein Verfahren zur Aufreinigung von Methacrylsäure, welches dadurch gekennzeichnet ist, dass die durch eine Umsetzung von Methacrylamid mit Wasser erhaltene Reaktionsmischung durch Mischen mit einem wässrigen Medium gekühlt und anschließend in einen Phasentrenner geleitet wird.

Durch diese Maßnahmen gelingt es überraschend ein Verfahren zur Herstellung bzw. zur Aufreinigung von Methacrylsäure bereitzustellen, welches ein besonders hervorragendes Eigenschaftsprofil aufweist. Überraschend gelingt durch das erfindungsgemäße Verfahren eine besonders einfache, sichere und kostengünstige Herstellung von Methacrylsäure. Hierbei kann unter anderem die Ausbeute, die Reinheit und die Standzeit der Anlage zur Herstellung Methacrylsäure erhöht werden. Darüber hinaus kann die Bildung von unerwünschten Nebenprodukten durch das Verfahren der vorliegenden Erfindung minimiert werden. Bevorzugte Anlagen zur Durchführung des Verfahrens der vorliegenden Erfindung können auf nicht ohne weiteres vorhersehbare Weise einen Beitrag zu den genannten Vorteilen leisten.

Gemäß dem vorliegenden Verfahren wird Methacrylsäure durch Umsetzung von Methacrylamid mit Wasser erhalten. Diese Reaktion kann als Hydrolyse oder Verseifung bezeichnet werden.

Die Umsetzung kann chargenweise oder kontinuierlich, beispielsweise in einem Rohrreaktor oder einem Rührkesselreaktor erfolgen. Gemäß einem besonderen Aspekt der vorliegenden Erfindung kann die Umsetzung kontinuierlich in einem Rohrreaktor erfolgen. Die Begriffe "kontinuierlich" und "Rohrreaktor" sind in der Fachwelt bekannt. Unter einer kontinuierlichen Reaktion sind insbesondere Umsetzungen zu verstehen, bei denen über einen längeren Zeitraum Edukte zugefügt und Produkte aus dem Reaktionsgemisch entfernt werden. Rohrreaktoren umfassen mindestens einen röhrenförmigen Bereich, in dem die Reaktion erfolgen kann. Diese Reaktoren weisen üblich einen relativ einfachen Aufbau auf, so dass die Investitionskosten vergleichsweise gering sind.

Vorzugsweise kann innerhalb des Rohrreaktors ein Druckunterschied in Flussrichtung der Reaktionsmischung vorhanden sein. Dieser Druckunterschied kann unter anderem durch verschiedene Teilbereiche innerhalb des Rohrreaktors erzielt werden, die beispielsweise durch Ventile gegeneinander abgegrenzt sind. Gemäß einer bevorzugten Ausführungsform des vorliegenden Verfahrens kann dieses Merkmal durch hydrostatischen Druck erzeugt werden, wobei die Reaktionsmischung in Flussrichtung einen Höhenunterschied durchläuft.

Der bevorzugt vorhandene Druckunterschied zwischen der Zuleitung der Edukte und der Ableitung der Produkte des Rohrreaktors ist an sich nicht kritisch, wobei sich überraschende Vorteile bei einem hohen Druckunterschied deutlich zeigen. Allerdings ist ein hoher Druckunterschied vielfach mit hohen Investitionskosten verbunden. Unerwartete Vorteile können insbesondere erzielt werden, falls der Druckunterschied mindestens 0,5, besonders bevorzugt mindestens 1 bar und ganz besonders bevorzugt mindestens 1,5 bar beträgt. Der Druckgradient kann hierbei jede Form annehmen, wie zum Beispiel eine Stufenform. Besonders bevorzugt sind jedoch Verfahren, bei denen der Druck kontinuierlich verändert wird. Dies kann insbesondere durch Rohrreaktoren bewirkt werden, deren Achse, die durch die Flussrichtung der Reaktionsmischung definiert wird, eine Neigung gegenüber der Erdoberfläche aufweisen. Bevorzugt beträgt der Neigungswinkel etwa 90°, d.h. der Rohrreaktor ist in Bezug auf die Erdoberfläche im Wesentlichen senkrecht ausgerichtet, wobei kleinere Abweichungen, beispielsweise von weniger als 10°, bevorzugt weniger als 5°, vielfach toleriert werden können.

Gemäß einer besonders zweckmäßigen Ausgestaltung können die Edukte zu Beginn der Reaktion bei einem hohen Druck und zu Ende der Reaktion bei einem geringeren Druck umgesetzt werden. Diese Abwandlung kann bevorzugt dadurch ausgestaltet werden, dass ein Rohrreaktor, der gegenüber der Erdoberfläche einen Neigungswinkel aufweist, von unten nach oben durchströmt wird, so dass die Flussrichtung eine Richtungskomponente aufweist, die senkrecht zur Erdoberfläche ausrichtet ist.

Die Umsetzung kann bei Über- oder Unterdruck erfolgen. Überraschende Vorteile hinsichtlich der Ausbeute und der Reinheit des Produkts sowie der Standzeiten der Anlage können insbesondere dadurch erzielt werden, dass die Reaktion bei einem Druck im Bereich von 1 bar bis 8 bar, vorzugsweise 1,5 bar bis 6 bar und besonders bevorzugt im Bereich von 2 bis 5 bar erfolgt.

Zweckmäßig kann die Umsetzung zu Beginn der Reaktion bei einem Druck im Bereich von 1,5 bis 6 bar, insbesondere 2 bis 4,5 bar und besonders bevorzugt 2,5 bis 3,5 bar und zu Ende der Reaktion bei einem Druck im Bereich von 1 bis 5 bar, besonders bevorzugt 1,5 bis 4 und ganz besonders bevorzugt 2 bis 3 bar durchgeführt werden.
Die Reaktionstemperatur kann ebenfalls in einem weiten Bereich liegen. Vorteile, die an sich nicht vorhersehbar sind, lassen sich jedoch beispielsweise in Bezug auf die Ausbeute, die Reaktionsgeschwindigkeit und die Standzeit der Anlage dadurch erzielen, dass die Umsetzung bei einer Temperatur im Bereich von 90°C bis 150°C, bevorzugt 100°C bis 140°C erfolgt.

Die zuvor dargelegten Verbesserungen können überraschend dadurch zusätzlich gesteigert werden, dass die Edukte zu Beginn der Reaktion bei einer geringeren Temperatur als zu Ende der Reaktion umgesetzt werden. Zweckmäßig kann die Temperatur zu Ende der Reaktion mindestens 5°C höher, bevorzugt mindestens höher 10°C sein als zu Beginn der Reaktion. Vorzugsweise ist die Temperatur zu Ende der Reaktion höchstens 25°C höher, bevorzugt höchstens 20°C höher als zu Beginn der Reaktion. Beispielsweise kann die Umsetzung zu Beginn der Reaktion bei einer Temperatur im Bereich von 100°C bis 130°C, vorzugsweise im Bereich 105°C bis 125°C und ganz besonders bevorzugt im Bereich von 110°C bis 120°C liegen. Gegen Ende der Reaktion sind Temperaturen im Bereich von 110°C bis 150°C besonders bevorzugt. Gemäß einer besonderen Ausgestaltung kann die maximal erreichte Temperatur höchstens 160°C, besonders bevorzugt höchstens 150°C und ganz besonders bevorzugt höchstens 135°C betragen. Zweckmäßig kann die Temperatur an mindestens zwei Bereichen des Reaktors gemessen und geregelt werden.

Neben den Edukten kann die Reaktionsmischung Additive umfassen, die an sich in der Fachwelt bekannt sind. Hierzu gehören insbesondere Katalysatoren, wie zum Beispiel Säuren, und Stabilisatoren, die eine Polymerisation der ungesättigten Verbindungen verhindern.

Vorzugsweise kann die Reaktion mit Säure katalysiert werden, wobei besonders bevorzugt Schwefelsäure eingesetzt werden kann. Die Schwefelsäure kann der Reaktionsmischung zusätzlich beigefügt werden. Darüber hinaus kann die Schwefelsäure bereits in einem der Edukte, beispielsweise im Methacrylamid und/oder Wasser enthalten sein. Der pH-Wert des eingesetzten Gemisches kann vorzugsweise im Bereich von 1 bis 7, besonders bevorzugt im Bereich von 1 bis 2 liegen.

Um eine unerwünschte Polymerisation der ungesättigten Verbindungen zu verhindern, können bei der Umsetzung Polymerisationsinhibitoren eingesetzt werden. Diese Verbindungen, wie beispielsweise Hydrochinone, Hydrochinonether, wie Hydrochinonmonomethylether oder Di-tert-butylbrenzcatechin, Phenothiazin, N,N'-(Diphenyl)-p-phenylendiamin, 4-Hydroxy-2,2,6,6-tetramethylpiperidin-1-oxyl, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Die Beschickung des Rohrreaktors mit Wasser kann grundsätzlich dahingehend erfolgen, dass Wasser aus einer beliebigen Quelle dem Reaktor zugeführt wird, sofern dieses Wasser keine Inhaltsstoffe aufweist, die die Verseifungsreaktion oder die nachfolgenden Prozeßstufen nachteilig beeinflussen könnten. Beispielsweise kann dem Reaktor VE-Wasser oder Brunnenwasser zugeführt werden. Es ist jedoch ebenfalls möglich, dem Reaktor ein Gemisch aus Wasser und organischen Verbindungen zuzuführen, wie sie beispielsweise bei der Reinigung von Methacrylsäure anfallen. Im Rahmen einer bevorzugten Ausführungsform des hier vorgestellten Verfahrens wird der Reaktor zumindest anteilig mit einem Gemisch aus Wasser und solchen organischen Verbindungen beschickt. Das molare Verhältnis von Wasser zu Methacrylamid in der eingesetzten Reaktionsmischung kann beispielsweise im Bereich von 7:1 bis 1:1, besonders bevorzugt 5,5:1 bis 4:1 liegen.

Vorzugsweise kann ein Gemisch eingesetzt werden, welches
10 bis 40 Gew.-%, besonders bevorzugt 20 bis 30 Gew.-% Methacrylsäureamid, 20 bis 50 Gew.-%, besonders bevorzugt 26 bis 32 Gew.-% Wasser, 30 bis 65 Gew.-%, besonders bevorzugt 40 bis 52 Gew.-% Schwefelsäure und 0 bis 5 Gew.-%, besonders bevorzugt 0,1 bis 4 Gew.-% zusätzliche Additive umfasst.

Überraschende Vorteile lassen sich insbesondere bei einer Verweilzeit im Bereich von 10 Minuten bis 2 Stunden, besondere bevorzugt 20 Minuten bis 1 Stunde erzielen.

Für den erfindungsgemäßen Erfolg ist es wesentlich, dass die durch die Umsetzung erhaltene Reaktionsmischung durch Mischen mit einem wässrigen Medium gekühlt und anschließend in einen Phasentrenner geleitet wird. Hierdurch gelingt es überraschend die Standzeit der Anlage zu erhöhen, wobei auch weitere Vorteile erzielt werden. Insbesondere kann durch diese Ausgestaltung die Ausbeute an Methacrylsäure erhöht und die Bildung von Nebenprodukten, insbesondere festen Bestandteilen in der Reaktionsmischung minimiert werden.

Vorzugsweise weist das wässrige Medium, durch das die erhaltene Reaktionsmischung gekühlt wird, eine Temperatur im Bereich von 20 °C bis 80 °C, besonders bevorzugt 40 bis 75°C auf. Das Volumenverhältnis von der durch die Umsetzung erhaltenen Reaktionsmischung zu wässrigem Medium kann im Bereich von 2:1 bis 1:5, besonders bevorzugt 1:1 bis 1:3 liegen.

Das zur Abkühlung einzusetzende wässrige Medium kann dem Produktionsprozess frisch zugeführt werden. Das wässrige Medium kann beispielsweise aus Wasser bestehen oder weitere Bestandteile enthalten, die jedoch keinen nachteiligen Einfluss auf die Reaktionsmischung ausüben sollten. Besonders zweckmäßig ist die Verwendung eines wässrigen Mediums, das durch Separation der durch die Umsetzung erhaltenen Reaktionsmischung gewonnen wird. Durch diese Maßnahme können die Kosten erheblich gesenkt werden. Technisch besonders günstig kann das wässrige Medium aus dem Phasentrenner abgeleitet werden. Diese Ausgestaltung ist durch den überraschenden Befund möglich, dass die bei einer Separation erhältliche wässrige Phase vielfach relativ geringe Anteile an organischen Bestandteilen, insbesondere an festen Nebenprodukten enthält, wobei diese Anteile durch Reinigung oder durch ein geeignetes Volumen eines ersten Phasentrenners und das hiermit verbundene Rücklaufverhältnis weiter minimiert werden können.

Zur Durchführung des erfindungsgemäßen Verfahrens kann eine Anlage genau einen Phasentrenner aufweisen, in dem die erhaltene Reaktionsmischung in eine organische und eine wässrige Phase aufgetrennt wird. Vorzugsweise weist eine Anlage zwei oder mehr Phasentrenner auf, so dass die Reaktionsmischung von einem ersten Phasentrenner in einen zweiten Phasentrenner eingeleitet wird.

Durch diese Ausgestaltung des Verfahrens kann der Kühlkreislauf auf besonders einfache und damit wartungsarme Weise von dem Austrag der anorganischen Phase entkoppelt werden. Unter anderem kann der Austrag der im zweiten Phasentrenner enthaltenen anorganischen Phase gesteuert werden, um weitere Verbesserungen der Standzeit der Anlage zu erzielen. Die Steuerung des Austrags der anorganischen Phase kann über eine Differenzdruckmessung und/oder eine kapazitive Messung erfolgen.

Der Austragsstrom kann durch einen Sieb geleitet werden, so dass gröbere Partikel zurückgehalten werden. Die zunächst zurückgehaltenen gröberen Partikel können vorzugsweise mechanisch zerkleinert werden. Diese Zerkleinerung kann beispielsweise durch turbulente Strömungen erfolgen. Diese Ausgestaltung trägt zu einer weiteren Verbesserung eines störungsfreien Betriebs der Anlage bei, wobei diese Ausgestaltung insbesondere durch die spezielle Kühlung der Reaktionsmischung ermöglicht wird, da hierdurch die Nebenproduktbildung so stark minimiert werden kann, dass lediglich geringe Mengen an gröberen Partikeln zerkleinert werden müssen.

Organische Verbindungen, die in der abgetrennten wässrigen Phase enthalten sind, können gemäß einer besonderen Abwandlung des erfindungsgemäßen Verfahrens isoliert werden. Hierzu kann die abgetrennte wässrige Phase zunächst in einen Kessel eingeleitet werden, der mit Dampf beaufschlagt wird, um die noch in der wässrigen Phase vorhandenen organischen Bestandteile auszutreiben. Die ausgetriebenen organischen Bestandteile, die einen hohen Anteil an Methacrylsäure aufweisen, können aufgereinigt und wiedergewonnen werden.

Gemäß einer besonderen Ausgestaltung kann die organische Phase der separierten Reaktionsmischung durch eine zweistufige Destillation aufgereinigt werden. Bevorzugt werden zunächst niedrig siedende Nebenprodukte aus der organischen Phase abgetrennt, so dass rohe Methacrylsäure aus dem Sumpf der ersten Destille in eine zweite Destille überführt wird.

Zur Abtrennung von niedrig siedenden Bestandteilen der organischen Phase wird die rohe Methacrylsäure vorzugsweise in die obere Hälfte einer Destillationskolonne zugeführt. Der Kolonnensumpf wird bevorzugt derart beheizt, dass eine Wandtemperatur von etwa 50 bis etwa 120°C erreicht wird. Die Reinigung wird üblich unter Vakuum durchgeführt. Der Druck innerhalb der Kolonne beträgt vorzugsweise etwa 40 bis etwa 300 mbar.

Am Kolonnenkopf werden die niedrigsiedenden Bestandteile abgenommen. Insbesondere können dieses beispielsweise Ether, Aceton und Methylformiat sein. Die Brüden werden anschließend über einen oder mehrere Wärmetauscher kondensiert. Dabei hat es sich beispielsweise in einigen Fällen bewährt, zunächst eine Kondensation über zwei in Serie geschaltete, mit Wasser gekühlte Wärmetauscher durchzuführen. Es ist jedoch ebenso möglich, an dieser Stelle auch nur einen Wärmetauscher einzusetzen. Die Wärmetauscher werden vorzugsweise zur Erhöhung der Flussgeschwindigkeit und um vollständige Oberflächenbenetzung zu erzielen, in senkrechtem Zustand betrieben. Dem wassergekühlten Wärmetauscher oder den wassergekühlten Wärmetauschern nachgeschaltet, kann ein solegekühlter Wärmetauscher sein, es ist jedoch auch möglich, eine Kaskade von zwei oder mehr solegekühlten Wärmetauschern nachzuschalten. Gemäß einer weiteren Ausgestaltung der Vorrichtung können niedrig siedende Bestandteile auch über einen Niedersiederauslass abgeleitet werden. In der Kaskade von Wärmetauschern werden die Brüden kondensiert, mit Stabilisator versehen und beispielsweise einem Phasentrenner zugeführt. Da die Brüden auch Wasser enthalten können, wird eine evtl. anfallende wässrige Phase entsorgt oder einer weiteren Verwertung zugeführt. Als weitere Verwertung bietet sich beispielsweise die Rückführung in eine Verseifungsreaktion an.

Die abgetrennte organische Phase kann teilweise als Rückfluss in den Kolonnenkopf eingespeist werden. Ein Teil der organischen Phase kann wiederum zum Besprühen der Wärmetauscherköpfe und des Kolonnenkopfes eingesetzt werden. Da es sich bei der abgetrennten organischen Phase um eine Phase handelt, die mit Stabilisator versetzt ist, lässt sich so wirksam einerseits die Ausbildung von Ruhezonen verhindern. Andererseits bewirkt die Gegenwart des Stabilisators eine weitere Unterbindung der Polymerisationsneigung der abgetrennten Brüden.

Der aus den Wärmetauschern erhaltene Kondensatstrom wird darüber hinaus vorzugsweise derart mit demineralisiertem Wasser versetzt, dass im Phasentrenner eine ausreichende Abscheidwirkung erzielt werden kann.

Die nach der Kondensation in der Wärmetauscherkaskade verbleibenden, gasförmigen Verbindungen können, vorzugsweise mittel Dampfstrahler als Unterdruckerzeuger, nochmals über einen oder mehrere weitere Wärmetauscher einer Kondensation unterzogen werden. Es hat sich dabei unter ökonomischen Gesichtspunkten als vorteilhaft herausgestellt, wenn im Rahmen einer solchen Nachkondensation nicht nur die gasförmigen Stoffe aus der Vorreinigung kondensiert werden. So ist es beispielsweise möglich, einer derartigen Nachkondensation weitere gasförmige Stoffe zuzuführen, wie sie sich aus der Hauptreinigung von Methacrylsäure ergeben. Der Vorteil einer derartigen Verfahrensweise liegt beispielsweise darin, dass so ein Anteil an Methacrylsäure, der im Rahmen der Hauptreinigungsstufe nicht kondensiert wurde, im Rahmen der Vorreinigung nochmals über den Phasenabscheider in die Reinigungskolonne überführt werden kann. So wird beispielsweise gewährleistet, dass eine Ausbeutemaximierung stattfinden kann, und möglichst geringe Verluste an Methacrylsäure auftreten. Außerdem kann durch die geeignete Wahl der Auslegung und des Betriebs dieser weiteren Wärmetauscher die Zusammensetzung des diese Wärmetauscher verlassenden Abgases, insbesondere der Gehalt an Leichtsiedern, eingestellt werden.

Zur Feinreinigung der Methacrylsäure wird die rohe, vorgereinigte Methacrylsäure einer erneuten Destillation unterzogen. Dabei wird die rohe Methacrylsäure mit Hilfe einer Destillationskolonne von den hochsiedenden Bestandteilen befreit und so eine Reinmethacrylsäure erhalten.

Die Destillationskolonne kann grundsätzlich einer beliebigen, dem Fachmann geeignet erscheinenden Ausführung entsprechen. Es hat sich jedoch für die Reinheit des erhaltenen Produkts in vielen Fällen als vorteilhaft herausgestellt, wenn die Destillationskolonne mit einer oder mehreren Packungen betrieben wird, die den folgenden Vorgaben etwa entspricht:
Zum einen sollen sich in den Kolonnen genauso wie in den anderen mit Methacrylsäure durchströmten Leitungen möglichst wenig so genannte "Toträume" bilden. Die Toträume führen zu einer vergleichsweise langen Verweilzeit der Methacrylsäure, die deren Polymerisation begünstigen. Weiterhin wurde überraschend festgestellt, dass Toträume vielfach nicht in ausreichendem Maß von optional eingesetzten Stabilisatoren oder Stabilisatorgemischen beaufschlagt werden. Die Polymerisation von Methacrylsäure führt wiederum zu kostspieligen Produktionsunterbrechungen und Reinigungen der entsprechenden mit Polymer zugesetzten Teile. Der Bildung von Toträumen kann unter anderem dadurch begegnet werden, dass sowohl durch Auslegung als auch durch eine geeignete Betriebsweise der Kolonnen, diese stets mit einer ausreichenden Menge von Flüssigkeit beladen werden, so dass eine ständige Umspülung der Kolonnen und insbesondere der Kolonneneinbauten wie Packungen erreicht wird. So können die Kolonnen Sprüheinrichtungen besitzen, die zum Besprühen der Kolonneneinbauten ausgelegt sind. Weiterhin können die Kolonneneinbauten untereinander so verbunden werden dass keine Toträume entstehen. Dies wird realisiert durch Ablauföffnungen oder unterbrochene Haftnähte wie im Folgenden beschreiben realisiert werden. Derartige Haftnähte weisen mindestens etwa 2, vorzugsweise mindestens etwa 5 und besonders bevorzugt mindestens etwa 10 Unterbrechungen auf 1 m Haftnahtlänge auf. Die Länge dieser Unterbrechungen kann so gewählt sein, dass diese mindestens etwa 10, vorzugsweise mindestens etwa 20 und besonders bevorzugt mindestens etwa 50 %, im allgemeinen aber nicht mehr als 95 % der Haftnahtlänge ausmachen. Eine andere bauliche Maßnahme kann darin liegen, dass in den Kolonneninnenbereichen, insbesondere die mit der Methacrylsäure in Kontakt kommen, weniger als etwa 50 %, vorzugsweise weniger als etwa 25 % und besonders bevorzugt weniger als etwa 10 % aller Flächen, insbesondere von Kolonneneinbauten, horizontal verlaufen. So können beispielsweise die in das Innere der Kolonne mündenden Stutzen konisch bzw. mit schrägen Flächen ausgestaltet sein. Ferner kann eine Maßnahme darin bestehen, die während des Betriebs der Kolonne im Kolonnensumpf befindliche Menge flüssiger Methacrylsäure so gering wie möglich zu halten und anderseits eine Überhitzung dieser Menge trotz moderaten Temperaturen und großen Verdampfungsflächen während des Verdampfen zu vermeiden. Hierbei kann es vorteilhaft sein, dass die Flüssigkeitsmenge im Kolonnensumpf im Bereich von etwa 0,1 bis 15 % und vorzugsweise etwa 1 bis 10 % der Gesamtmenge an Methacrylsäure in der Kolonne ausmacht.

Im Rahmen der Reinigung der Methacrylsäure werden deren hochsiedende Bestandteile durch Destillation vom Produkt getrennt. Vorzugsweise beträgt die Sumpftemperatur etwa 50 bis etwa 80 °C, insbesondere etwa 60 bis etwa 75 °C bei Wandtemperatur von weniger als etwa 120 °C.

Das im Kolonnensumpf anfallende Material wird vorzugsweise kontinuierlich abgeführt und über einen Wärmetauscher oder eine Kaskade von mehreren Wärmetauschern auf eine Temperatur in einem Bereich von etwa 40 bis etwa 80°C, vorzugsweise etwa 40 bis etwa 60°C und besonders bevorzugt in einem Bereich von etwa 50 bis 60°C abgekühlt.

Zur Verbesserung der Ausbeute kann der Sumpf der zweiten Destille nochmals verdampft werden. Vorzugsweise kann dieser Sumpf der zweiten Destille mit einem Umlaufverdampfer verdampft und in die zweite Destille eingeleitet werden. Diese Ausgestaltung ist aus Kostengründen gegenüber einem Dünnschichtverdampfer bevorzugt. Allerdings sind Umlaufverdampfer jedoch wartungsintensiver, falls feste Nebenprodukte in der zu verdampfenden Mischung enthalten sind. Überraschend wird jedoch durch eine überraschend effektive Kühlung der Reaktionsmischung, die zu einer unerwartet geringen Nebenproduktbildung und einer wirksamen Minimierung der festen Bestandteile führt, ein Einsatz von Umlaufverdampfern möglich.

Am Kolonnenkopf wird die destillativ aufgereinigte Methacrylsäure entnommen und über einen Wärmetauscher oder eine Kaskade von zwei oder mehr Wärmetauschern gekühlt. Dabei kann die Wärme der Brüden durch wassergekühlte Wärmetauscher oder durch solegekühlte Wärmetauscher oder durch eine Kombination aus beidem abgeführt werden. Es hat sich in einigen Fällen bewährt, wenn die Brüden aus der Destillationskolonne in zwei oder mehr parallel geschaltete Wärmetauscher überführt werden, die mittels Wasserkühlung betrieben werden. Die nicht kondensierten Anteile aus den wassergekühlten Wärmetauschern können beispielsweise in einen solegekühlten Wärmetauscher oder eine Kaskade von zwei oder mehr solegekühlten Wärmetauschern eingeleitet werden, die seriell oder parallel angeordnet sein können. Gemäß einer weiteren Ausgestaltung der Vorrichtung können niedrig siedende Bestandteile auch über einen Niedersiederauslass abgeleitet werden. Die aus den Wärmetauschern erhältlichen Kondensate werden in einen Sammelbehälter eingeleitet und mittels einer Pumpe über einen weiteren Wärmetauscher oder eine Kaskade von zwei oder mehr weiteren Wärmetauschern einem Pufferbehälter zugeführt. Der Kondensatstrom wird dabei beispielsweise über eine Kaskade von einem oder zwei wassergekühlten Wärmetauschern und einem oder zwei solegekühlten Wärmetauschern auf eine Temperatur in einem Bereich von etwa 18 bis etwa 50°C, vorzugsweise etwa 18 bis etwa 40°C und besonders bevorzugt in einem Bereich von etwa 18 bis 30°C heruntergekühlt.

Dem Kondensatstrom wird ein Teilstrom entnommen, der über den Kolonnenkopf in die Destillationskolonne zurückgeführt wird. Die Einspeisung des Kondensatstromes in den Kolonnenkopf kann dabei grundsätzlich auf beliebige Weise z.B. über Verteiler erfolgen. Es kann jedoch vorteilhaft sein, wenn ein Teil des Kondensatstromes oberhalb des Kolonnenkopfes in die Brüdenleitung eingespeist, beispielsweise eingesprüht, wird. Weiterhin ist es bevorzugt, dass mit dieser Einspeisung Stabilisator in den Kolonnenkopf eingetragen wird.

Ein weiterer Teilstrom des zur Rückführung in die Kolonne vorgesehenen Kondensates kann beispielsweise vor Einbringung in die Brüdenleitung abgezweigt und direkt in den Kolonnenkopf eingebracht werden. Auch hier ist es bevorzugt, dass mit dieser Einspeisung Stabilisator in den Kolonnenkopf eingetragen wird. Die Einbringung in den Kolonnenkopf kann dabei beispielsweise dergestalt geschehen, dass das Innere des Kolonnenkopfes mit dem Kondensat so besprüht wird, dass sich keine Ruhezonen im Kolonnenkopf ausbilden können, an denen eine Polymerisation der Methacrylsäure erfolgen kann. Es kann darüber hinaus vorteilhaft sein, wenn einem Kondensatteilstrom, der in die Kolonne zurückgeführt wird, ein Stabilisator zur Verhinderung von Polymerisation zugefügt wird. Dies kann beispielsweise dadurch geschehen, dass dem zum Besprühen des Kolonnenkopfes vorgesehenen Kondensatteilstrom eine entsprechende Menge an Polymerisationsinhibitor als Stabilisator zugefügt wird. Dabei hat es sich in einigen Fällen als vorteilhaft erwiesen, wenn der Kondensatteilstrom nach der Zugabe des Stabilisators, aber vor dem Eintritt in den Kolonnenkopf eine geeignete Mischvorrichtung, vorzugsweise einen Statikmischer durchläuft, um eine möglichst uniforme Verteilung des Stabilisators im Kondensatteilstrom zu erzielen.

Die im Rahmen des Reinigungsverfahrens anfallenden, nicht kondensierbaren gasförmigen Stoffe werden beispielsweise der Entsorgung zugeführt.

Die Anlage zur Durchführung des vorliegenden Verfahrens kann bekannte Reaktoren zur Umsetzung von Amiden mit Wasser aufweisen. Hierzu gehören insbesondere Rührkesselreaktoren sowie Rohrreaktoren. Vorzugsweise wird ein im Wesentlichen vertikal ausgerichteter Rohrreaktor eingesetzt. Hierdurch können überraschende Vorteile hinsichtlich der Standzeiten der Anlage sowie der Ausbeute und Produktreinheit erzielt werden.

Vorzugsweise weist der Rohrreaktor mindestens zwei, besonders bevorzugt mindestens drei Wärmetauscher auf, über die die Reaktionstemperatur auf ein gewünschtes Profil eingestellt werden kann.

Die Edukte können über eine Pumpe in den Rohrreaktor eingeleitet werden. Zur Vermeidung von wartungsbedingten Betriebsunterbrechungen können auch zwei oder mehr Pumpen vorgesehen sein, die parallel geschaltet werden können. Das Mischen der Edukte kann zweckmäßig in Strömungsrichtung gesehen vor den Pumpen erfolgen, wobei die Anlage besonders bevorzugt in dem Bereich zwischen den Pumpen und dem Rohrreaktor keine weiteren Einbauten zum Mischen aufweist. Durch diese Maßnahmen können überraschende Vorteile hinsichtlich der Betriebssicherheit und der Standzeiten der Anlage sowie in Bezug auf die Ausbeute und die Reinheit des Produkts erzielt werden.

Eine bevorzugte Anlage zur Durchführung des Verfahrens gemäß der vorliegenden Erfindung weist mindestens einen Phasentrenner auf, wobei der Phasentrenner durch eine Rückführung mit der Zuleitung verbunden ist, durch die die Reaktionsmischung in den Phasentrenner geleitet wird. Eine derartige Anlage ist ebenfalls Gegenstand der vorliegenden Erfindung.

Vorzugsweise kann die Rückführung, über die der Phasentrenner mit der Zuleitung verbunden ist, durch die die Reaktionsmischung in den Phasentrenner geleitet wird, mit einem Wärmetauscher ausgestattet sein. Hierdurch kann die zurückgeführte Phase temperiert werden.

Die Anlage kann ein, zwei oder mehr Phasentrenner aufweisen, wobei Anlagen mit mindestens zwei Phasentrennern bevorzugt sind. Zweckmäßig kann der erste Phasentrenner durch eine Rückführung mit der Zuleitung verbunden sein, durch die die Reaktionsmischung in den Phasentrenner geleitet wird. Der zweite Phasentrenner umfasst bevorzugt eine Vorrichtung zur Steuerung des anorganischen Austrags. Vorzugsweise weist der zweite Phasentrenner ein größeres Volumen auf, als der erste Phasentrenner. Der zweite Phasentrenner kann mindestens eine Entnahmestelle umfassen, an der mindestens ein Sieb vorgesehen ist, das gröbere Partikel zurückhält. Hierbei ist die Entnahmestelle bevorzugt so modifiziert, dass die vom Sieb zurückgehaltenen gröberen Partikel mechanisch zerkleinert werden. Die mechanische Zerkleinerung kann insbesondere durch eine turbulente Strömung bewirkt werden, wobei die hierdurch erzielte Reibung der Partikel an dem Sieb zu einer Zerkleinerung führt. Beispielsweise kann das Sieb in Form eines mit Bohrungen versehenen Einsteckrohres ausgestaltet sein.

Die Komponenten der Anlage, die mit Säure in Berührung kommen, insbesondere der Rohrreaktor, die Pumpen und die Phasentrenner sind bevorzugt aus einem säureresistenten Metall, beispielsweise Zirkonium, Tantal, Titan oder rostfreien Stahl oder einem beschichteten Metall, das beispielsweise eine Emailschicht oder eine Zirkonschicht aufweist, aufgebaut. Weiterhin können auch Kunststoffe, beispielsweise PTFE ummantelte Bauteile, graphitisierte Komponenten oder Werkstücke aus Graphit, insbesondere in Pumpen eingesetzt werden.

Das zur Herstellung von Methacrylsäure eingesetzte Methacrylamid kann vorzugsweise durch das so genannte ACH-Verfahren erhalten werden. Ausgehend von Blausäure und Aceton wird in einem ersten Schritt Acetoncyanhydrin hergestellt, welches anschließend zum Methacrylamid umgesetzt wird. Diese Schritte sind unter anderem in US 7,253,307, EP-A-1 666 451 und DE 102006058251.9 dargestellt.

Das Vorstehende wird nunmehr anhand von nicht limitierenden Zeichnungen beispielhaft erläutert. Die in den Figuren beschriebene Anlage bezieht sich vorliegend auf eine Anlage zur Herstellung von Methacrylsäure. Es zeigen:
- Fig. 1:: schematisch eine Anlage zur Umsetzung von Methacrylamid mit Wasser,
- Fig. 2:: schematisch eine Anlage zur Vorreinigung der Methacrylsäure,
- Fig. 3:: schematisch eine Feinreinigungsanlage der Methacrylsäure und
- Fig. 4:: schematisch eine Anlage zum Strippen der wässrigen Phase, die aus der Anlage zur Hydrolyse des Methacrylamids erhalten wird.

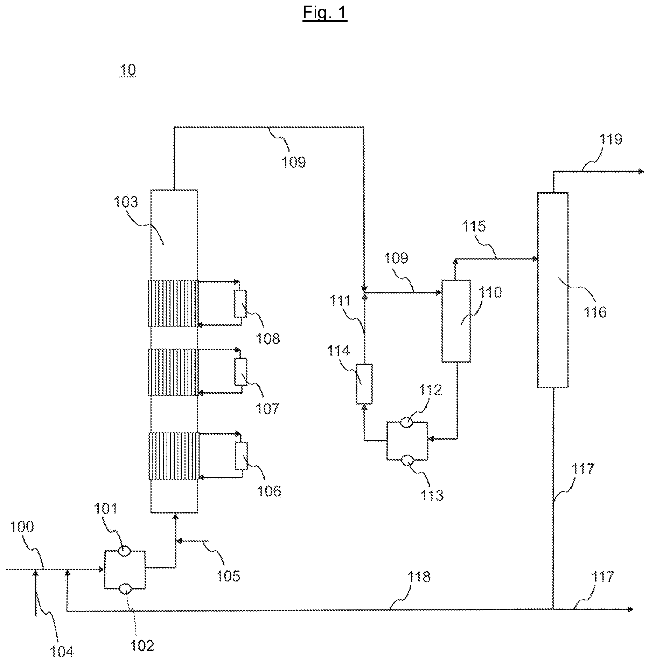

In Figur 1 ist eine bevorzugte Ausführungsform einer Anlage 10 dargestellt, in welchem die Umsetzung des Methacrylamids mit Wasser erfolgt. Über Leitungen 100 wird das Amid, von den Pumpen 101 und 102 gefördert, in den Rohrreaktor 103 geleitet. Das zur Umsetzung benötigte Wasser kann über eine externe Quelle in Leitung 100 eingespeist werden, die in Figur 1 über Leitung 104 mit Leitung 100 verbunden ist. Weiterhin kann zumindest ein Teil des Wassers aus einem später beschriebenen Phasentrenner 116 über Leitung 118 in Leitung 100 eingeleitet werden. Hierbei kann die Zuführung, in Flussrichtung gesehen, vor den Pumpen 101 und 102 erfolgen. Die Pumpen 101 und 102 sind parallel geschaltet. Durch eine parallele Schaltung der Pumpen 101 und 102 kann die Betriebssicherheit erhöht werden. Zur Stabilisierung der Redaktionsmischung können Stabilisatoren durch Leitung 105 beigefügt werden.

Der Rohrreaktor 103 kann vorzugsweise durch zwei, besonders bevorzugt drei Wärmetauscher 106, 107 und 108 temperiert werden, die vorliegend als integraler Bestandteil des Rohrreaktors ausgestaltet sind. Hierzu können Kühlflüssigkeiten eingesetzt werden, die durch Mäntel geleitet werden, die an verschiedenen Segmenten des Rohrreaktors vorgesehen sind.

Die umgesetzte Reaktionsmischung wird über Leitung 109 abgeleitet, die in einen ersten Phasentrenner 110 führt. Im ersten Phasentrenner wird ein Teil der wässrigen Phase abgetrennt. Dieser Teil der wässrigen Phase wird über Leitung 111 in die Leitung 109 zurückgeführt. Dieser Kreislauf wird durch die parallel geschalteten Pumpen 112, 113 aufrechterhalten. Zur Verbesserung der Kühlleistung der über Leitung 111 zurückgeführten wässrigen Phase weist die vorliegend dargestellte Ausführungsform einen Wärmetauscher 114 auf.

Die abgekühlte Reaktionsmischung wird über Leitung 115 aus dem ersten Phasentrenner 110 in den zweiten Phasentrenner 116 geleitet. Der zweite Phasentrenner 116 kann insbesondere eine Vorrichtung zur Steuerung des Austrags aufweisen, der in der vorliegenden Figur nicht dargestellt ist. Die wässrige Phase wird über Leitung 117 ausgetragen, wobei ein Teil der wässrigen Phase insbesondere durch Leitung 118 in die Amid führende Leitung 100 eingeleitet werden kann.

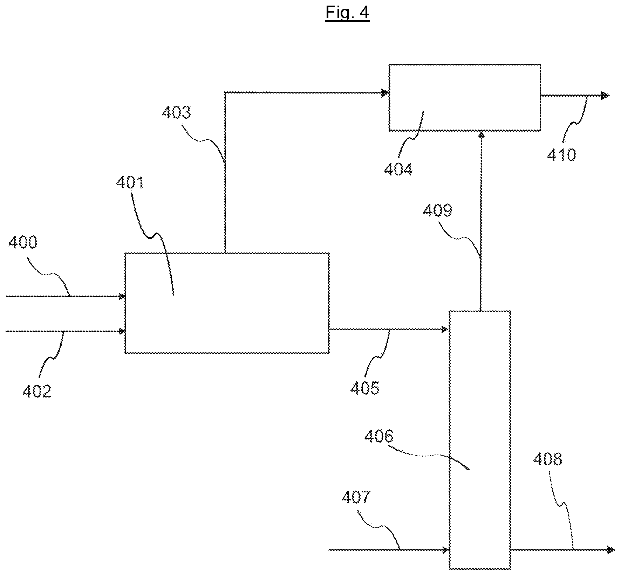

Der nicht zurückgeführte Teil der wässrigen Phase kann aufbereitet werden. Eine hierfür geeignete Anlage ist schematisch in Figur 4 dargestellt. Die organische Phase wird über Leitung 119 aus diesem Teil der Anlage abgeleitet. Vorzugsweise wird die organische Phase über einen zweistufigen Prozess aufgereinigt. Die in Figur 1 dargestellte Anlage kann an verschiedenen Stellen nicht dargestellte Gasabscheider aufweisen. Vorteilhaft kann insbesondere nach dem zweiten Phasentrenner 116 und vor der Destillationsanlage ein Gasabscheider vorgesehen sein.

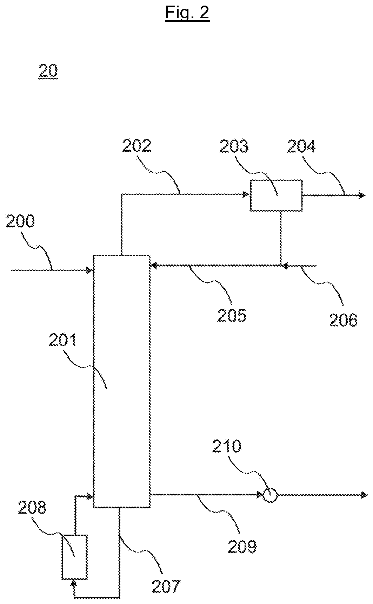

Figur 2 zeigt eine bevorzugte Destillationsanlage 20, über die die niedrig siedenden Bestandteile der organischen Phase, die aus der Redaktionsmischung erhalten werden kann, abgetrennt werden.

Eine organische Phase, die beispielsweise durch einen Austrag aus der in Figur 1 dargestellten Anlage 10 erhalten werden kann, wird über Leitung 200 in die Destillationskolonne 201 eingeleitet. Die Einleitung kann beispielsweise im Bereich des Kolonnenkopfes erfolgen. Die niedrig siedenden Bestandteile können aus dem Kopfbereich der Destillationskolonne 201 über Leitung 202 in einen Wärmetauscher 203 überführt werden. Im Wärmetauscher 203 werden die Brüden abgekühlt und über Leitung 204 ausgetragen, wobei ein Teil der kondensierten Stoffe in die Kolonne 201 durch Leitung 205 zurückgeführt werden können. Zur Stabilisierung kann in die Kolonne eine Stabilisatorzusammensetzung eingeleitet werden. Dies kann z.B. über den Zulauf 206 erfolgen, über den eine Stabilisatormischung in Leitung 205 eingeleitet werden kann.

Ein Teil des Kolonnensumpfs kann über Leitung 207 in einem Sumpfverdampfer 208 verdampft und in die Kolonne zurückgeleitet werden. Der Sumpf der Kolonne kann über Leitung 209 durch die Pumpe 210 aus dem System ausgetragen werden.

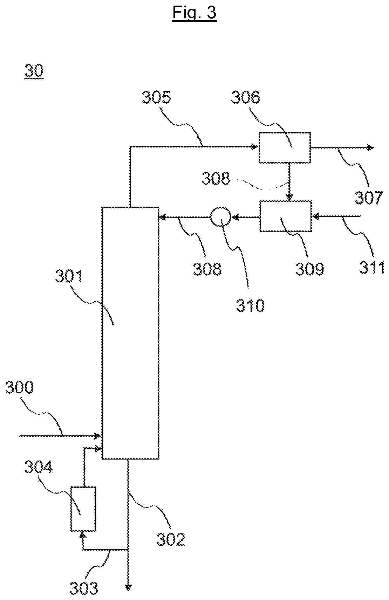

In Figur 3 ist eine bevorzugte Ausführungsform einer Destillationsanlage 30 dargestellt, mit der eine weitere Aufreinigung der im Sumpf der zuvor beschriebenen Destillationsanlage erhaltenen Methacrylsäure erfolgen kann.

Eine Methacrylsäure enthaltende Zusammensetzung kann über Leitung 300 in die Destillationskolonne 301 eingeleitet werden. Schwer siedende Bestandteile dieser Zusammensetzung werden über Leitung 302 durch den Sumpf aus der Kolonne ausgetragen. Ein Teil dieses Auftrages kann durch Leitung 303 in einem Sumpfverdampfer 304, der vorzugsweise als Umlaufverdampfer ausgestaltet ist, verdampft und in den Sumpf der Kolonne 301 eingeleitet werden.

Die Methacrylsäure enthaltenden Brüden werden über Leitung 305 der Kolonne entnommen und im Wärmeaustauscher 306 kondensiert. Die kondensierte Methacrylsäure wird durch Leitung 307 der Destillationsanlage 30 entnommen. Ein Teil der im Wärmeaustauscher 306 kondensierten Phase kann durch Leitung 308 in einen zweiten Wärmeaustauscher 309 eingeleitet und dort gekühlt werden. Über die Pumpe 310 kann die so gekühlte Phase in den Kopf der Destillationskolonne 301 eingeleitet werden. Zur Stabilisierung der in der Destillationskolonne 301 enthaltenen Zusammensetzung können Stabilisatoren eingesetzt werden. Eine Zuleitung der Stabilisatoren kann beispielsweise über Leitung 311 erfolgen, über die Stabilisatoren in den Wärmeaustauscher 309 eingeleitet werden können.
In Figur 4 ist schematisch eine bevorzugte Anlage zum Strippen der wässrigen Phase dargestellt, die aus der Anlage 10 zur Hydrolyse des Methacrylamids erhalten wird. Die wässrige Phase kann über Leitung 400 in einen Säurebehälter 401 eingeleitet werden, der über Zuführung 402 mit Dampf beaufschlagt wird. Die hierdurch freigesetzten gasförmigen Stoffe werden über Leitung 403 in einen Kondensator 404 abgeleitet. Die wässrige Phase wird über Leitung 405 in eine Kolonne 406 überführt, die über eine Zuführung 407 ebenfalls mit Dampf beaufschlagt wird. Der wässrige Austrag kann über Leitung 408 abgeleitet werden. Die Dampfphase wird über Leitung 409 in einen Kondensator 404 eingeleitet. Die kondensierte Phase wird über Leitung 410 der Anlage entnommen und kann unter anderem dem in Figur 1 dargestellten Verseifungsprozess zugeführt werden, da diese Phase einen hohen Anteil an Wasser und leichtflüchtigen organischen Bestandteilen, insbesondere an Methacrylsäure und Methacrylamid aufweist.

Nachfolgend soll die vorliegende Erfindung anhand eines Beispiels näher erläutert werden, ohne dass hierdurch eine Beschränkung erfolgen soll.

### Beispiel 1

In einer in Figur 1 dargelegten Anlage Methacrylsäure (MAS) hergestellt. Hierbei wird mit Rohrreaktor 103 (Reaktor; H=19,7 m, D=0,78 m, max. zul. Druck: 6 bar, max. zul. Temperatur: 200°C) ein nahezu störungsfreier Betrieb der Anlage über mehr als 6 Monate gewährleistet. Hierbei kann die Anlagenbelastung in weiten Bereichen variiert werden. Lasten bis zu 20 000 Jahrestonnen MAS mit einem Reinheitsgrad von mindestens 99,5 % (HPLC) werden erreicht.

## Patentansprüche

1. Verfahren zur Aufreinigung von Methacrylsäure, **dadurch gekennzeichnet, dass** die durch eine Umsetzung von Methacrylamid mit Wasser erhaltene Reaktionsmischung durch Mischen mit einem wässrigen Medium gekühlt und anschließend in einen Phasentrenner (110, 116) geleitet wird.

2. Verfahren gemäß Anspruch 1, **dadurch gekennzeichnet, dass** das wässrige Medium durch Separation der durch die Umsetzung erhaltenen Reaktionsmischung gewonnen wird.

3. Verfahren gemäß Anspruch 2, **dadurch gekennzeichnet, dass** das wässrige Medium aus dem Phasentrenner (110, 116) abgeleitet wird.

4. Verfahren gemäß mindestens einem der vorhergehenden Ansprüche, **dadurch gekennzeichnet, dass** das wässrige Medium eine Temperatur im Bereich von 20 °C bis 80 °C aufweist.

5. Verfahren gemäß mindestens einem der vorhergehenden Ansprüche, **dadurch gekennzeichnet, dass** die Reaktionsmischung von einem ersten Phasentrenner (110) in einen zweiten Phasentrenner (116) eingeleitet wird.

6. Verfahren gemäß Anspruch 5, **dadurch gekennzeichnet, dass** der Austrag der im zweiten Phasentrenner (116) enthaltenen anorganischen Phase gesteuert wird.

7. Verfahren gemäß Anspruch 6, **dadurch gekennzeichnet, dass** die Steuerung des Austrags der anorganischen Phase über eine Differenzdruckmessung und/oder eine kapazitive Messung erfolgt.

8. Verfahren gemäß mindestens einem der Ansprüche 5 bis 7, **dadurch gekennzeichnet, dass** der Austragsstrom durch einen Sieb geleitet wird, so dass gröbere Partikel zurückgehalten werden.

9. Verfahren gemäß Anspruch 8, **dadurch gekennzeichnet, dass** gröbere Partikel mechanisch zerkleinert werden.

10. Verfahren gemäß mindestens einem der vorhergehenden Ansprüche, **dadurch gekennzeichnet, dass** das Volumenverhältnis von der durch die Umsetzung erhaltenen Reaktionsmischung zu wässrigem Medium im Bereich von 2:1 bis 1:5 liegt.

11. Verfahren gemäß mindestens einem der vorhergehenden Ansprüche, **dadurch gekennzeichnet, dass** die Umsetzung bei einer Temperatur im Bereich von 90°C bis 150°C erfolgt.

12. Verfahren gemäß mindestens einem der vorhergehenden Ansprüche, **dadurch gekennzeichnet, dass** die Umsetzung bei einem Druck im Bereich von 1,5 bar bis 6 bar erfolgt.

13. Verfahren gemäß mindestens einem der vorhergehenden Ansprüche, **dadurch gekennzeichnet, dass** die Umsetzung in einem Rohrreaktor (103) durchgeführt wird, der von unten nach oben durchströmt wird.

14. Verfahren gemäß mindestens einem der vorhergehenden Ansprüche, **dadurch gekennzeichnet, dass** die Reaktion mit Säure katalysiert wird.

15. Verfahren gemäß mindestens einem der vorhergehenden Ansprüche, **dadurch gekennzeichnet, dass** das Reaktionsgemisch mindestens einen Stabilisator umfasst.

16. Verfahren gemäß mindestens einem der vorhergehenden Ansprüche, **dadurch gekennzeichnet, dass** ein Gemisch eingesetzt wird, welches
10 bis 40 Gew.-% Methacrylsäureamid,
20 bis 50 Gew.-% Wasser,
30 bis 65 Gew.-% Schwefelsäure und
0 bis 5 Gew.-% zusätzliche Additive umfasst.

17. Verfahren gemäß mindestens einem der vorhergehenden Ansprüche, **dadurch gekennzeichnet, dass** der pH-Wert des eingesetzten Gemisches im Bereich von 1 bis 2 liegt.

18. Verfahren gemäß mindestens einem der vorhergehenden Ansprüche, **dadurch gekennzeichnet, dass** die Verweilzeit im Bereich von 10 Minuten bis 2 Stunden liegt.

19. Verfahren gemäß mindestens einem der vorhergehenden Ansprüche, **dadurch gekennzeichnet, dass** die organische Phase der separierten Reaktionsmischung durch eine zweistufige Destillation aufgereinigt wird.

20. Verfahren gemäß Anspruch 19, **dadurch gekennzeichnet, dass** der Sumpf der zweiten Destille (301) mit einem Umlaufverdampfer (304) verdampft und in die zweite Destille eingeleitet wird.

## Claims

1. Process for purifying methacrylic acid, **characterized in that** the reaction mixture obtained by a reaction of methacrylamide with water is cooled by mixing with an aqueous medium and then passed into a phase separator (110, 116).

2. Process according to Claim 1, **characterized in that** the aqueous medium is obtained by separating the reaction mixture obtained by the reaction.

3. Process according to Claim 2, **characterized in that** the aqueous medium is discharged from the phase separator (110, 116).

4. Process according to at least one of the preceding claims, **characterized in that** the aqueous medium has a temperature in the range of 20°C to 80°C.

5. Process according to at least one of the preceding claims, **characterized in that** that the reaction mixture is introduced from a first phase separator (110) into a second phase separator (116).

6. Process according to Claim 5, **characterized in that** the discharge of the inorganic phase present in the second phase separator (116) is controlled.

7. Process according to Claim 6, **characterized in that** the discharge of the inorganic phase is controlled by means of a pressure differential measurement and/or a capacitive measurement.

8. Process according to at least one of Claims 5 to 7, **characterized in that** the discharge stream is passed through a screen such that relatively coarse particles are retained.

9. Process according to Claim 8, **characterized in that** relatively coarse particles are mechanically comminuted.

10. Process according to at least one of the preceding claims, **characterized in that** the volume ratio of the reaction mixture obtained by the reaction relative to aqueous medium is within the range of 2:1 to 1:5.

11. Process according to at least one of the preceding claims, **characterized in that** the reaction is effected at a temperature in the range from 90°C to 150°C.

12. Process according to at least one of the preceding claims, **characterized in that** the reaction is effected at a pressure in the range of 1.5 bar to 6 bar.

13. Process according to at least one of the preceding claims, **characterized in that** the reaction is carried out in a tubular reactor (103) which is flowed through from the bottom upward.

14. Process according to at least one of the preceding claims, **characterized in that** the reaction is catalysed with acid.

15. Process according to at least one of the preceding claims, **characterized in that** the reaction mixture comprises at least one stabilizer.

16. Process according to at least one of the preceding claims, **characterized in that** a mixture is used which comprises
10 to 40% by weight of methacrylamide,
20 to 50% by weight of water,
30 to 65% by weight of sulphuric acid and
0 to 5% by weight of additional additives.

17. Process according to at least one of the preceding claims, **characterized in that** the pH of the mixture used is in the range of 1 to 2.

18. Process according to at least one of the preceding claims, **characterized in that** the residence time is in the range of 10 minutes to 2 hours.

19. Process according to at least one of the preceding claims, **characterized in that** the organic phase of the separated reaction mixture is purified by a two-stage distillation.

20. Process according to Claim 19, **characterized in that** the bottoms of the second still (301) are evaporated with a circulation evaporator (304) and introduced into the second still.

## Revendications

1. Procédé de purification d'acide méthacrylique, **caractérisé en ce que** le mélange réactionnel obtenu par une réaction de méthacrylamide avec de l'eau est refroidi par mélange avec un milieu aqueux, puis conduit dans un séparateur de phases (110, 116).

2. Procédé selon la revendication 1, **caractérisé en ce que** le milieu aqueux est obtenu par séparation du mélange réactionnel obtenu par la réaction.

3. Procédé selon la revendication 2, **caractérisé en ce que** le milieu aqueux est déchargé du séparateur de phases (110, 116).

4. Procédé selon au moins l'une quelconque des revendications précédentes, **caractérisé en ce que** le milieu aqueux présente une température dans la plage allant de 20 °C à 80 °C.

5. Procédé selon au moins l'une quelconque des revendications précédentes, **caractérisé en ce que** le mélange réactionnel issu d'un premier séparateur de phases (110) est introduit dans un deuxième séparateur de phases (116).

6. Procédé selon la revendication 5, **caractérisé en ce que** la sortie de la phase inorganique contenue dans le deuxième séparateur de phases (116) est régulée.

7. Procédé selon la revendication 6, **caractérisé en ce que** la régulation de la sortie de la phase inorganique a lieu par une mesure de pression différentielle et/ou une mesure de capacité.

8. Procédé selon au moins l'une quelconque des revendications 5 à 7, **caractérisé en ce que** le courant de sortie est conduit au travers d'un tamis, de manière à retenir les particules plus grossières.

9. Procédé selon la revendication 8, **caractérisé en ce que** les particules plus grossières sont broyées mécaniquement.

10. Procédé selon au moins l'une quelconque des revendications précédentes, **caractérisé en ce que** le rapport en volume entre le mélange réactionnel obtenu par la réaction et le milieu aqueux se situe dans la plage allant de 2:1 à 1:5.

11. Procédé selon au moins l'une quelconque des revendications précédentes, **caractérisé en ce que** la réaction a lieu à une température dans la plage allant de 90 °C à 150 °C.

12. Procédé selon au moins l'une quelconque des revendications précédentes, **caractérisé en ce que** la réaction a lieu à une pression dans la plage allant de 1,5 bar à 6 bar.

13. Procédé selon au moins l'une quelconque des revendications précédentes, **caractérisé en ce que** la réaction est réalisée dans un réacteur tubulaire (103), qui est traversé du bas vers le haut.

14. Procédé selon au moins l'une quelconque des revendications précédentes, **caractérisé en ce que** la réaction est catalysée avec un acide.

15. Procédé selon au moins l'une quelconque des revendications précédentes, **caractérisé en ce que** le mélange réactionnel comprend au moins un stabilisateur.

16. Procédé selon au moins l'une quelconque des revendications précédentes, **caractérisé en ce qu'**un mélange qui comprend :
10 à 40 % en poids d'un amide de l'acide méthacrylique,
20 à 50 % en poids d'eau,
30 à 65 % en poids d'acide sulfurique et
0 à 5 % en poids d'additifs supplémentaires est utilisé.

17. Procédé selon au moins l'une quelconque des revendications précédentes, **caractérisé en ce que** le pH du mélange utilisé se situe dans la plage allant de 1 à 2.

18. Procédé selon au moins l'une quelconque des revendications précédentes, **caractérisé en ce que** le temps de séjour se situe dans la plage allant de 10 minutes à 2 heures.

19. Procédé selon au moins l'une quelconque des revendications précédentes, **caractérisé en ce que** la phase organique du mélange réactionnel séparé est purifiée par une distillation à deux étapes.

20. Procédé selon la revendication 19, **caractérisé en ce que** le fond de la deuxième distillation (301) est évaporé avec un évaporateur à circulation (304) et introduit dans la deuxième distillation.
